# EUROPEAN PATENT APPLICATION

(11) **EP 2 439 535 A1**
(43) Date of publication of application: **11.04.2012**
(21) Application number: 10186898.2
(22) Date of filing: 07.10.2010
(51) Int. Cl.: G01N 33/68

(54) **Diagnosis of diabetes related heart disease and GDF-15 and Troponin as predictors for the development of type 2 diabetes mellitus**

(71) Applicant: Hoffmann-La Roche AG, 4070 Basel (CH); Roche Diagnostics GmbH, 68305 Mannheim (DE)
(72) Inventor: The designation of the inventor has not yet been filed
(74) Representative: Huhn, Michael

(57) **Abstract**

The present invention relates to the field of in vitro diagnostics related to diabetes. In particular means and methods for the prediction of the risk of a patient to suffer from type 2 diabetes and for the diagnosis of its complication are disclosed. The invention employs cardiac troponins, GDF-15, natriuretic peptides, H-FABP and variants of the aforementioned peptides as biomarkers.

## Description

The present invention relates to the field of *in vitro* diagnostics related to diabetes. In particular means and methods for the prediction of the risk of a patient to suffer from type 2 diabetes and for the diagnosis of its complication are disclosed. The invention employs cardiac troponins, GDF-15, natriuretic peptides, H-FABP and variants of the aforementioned peptides as biomarkers.

Diabetes mellitus is an endemic disease which affects an increasing number of individuals worldwide. In fact, the number of individuals with diabetes was 171 million in 2000 and is believed to increase to 366 million individuals in 2030 (Dostalova et al., 2009, Europ. J. of Endocrinology, 161: 397-404). Among the various complications of diabetes are cardiac complications. Diabetes mellitus is associated with an increased prevalence of coronary artery disease. While some of the risk factors for coronary artery disease in diabetics are identical to those in subjects without diabetes, there are some risk factors specific for diabetics: they have more lipid in atherosclerotic plaques and the plaques appear to be more vulnerable. In addition to this, chronic heart failure is different in diabetes mellitus patients as compared to non-diabetics. Said differences are related to advanced glycosylation endproducts that have been related to diastolic dysfunction. Moreover, myocardial calcium handling, myocardial metabolism and coronary microcirculation are abnormal in patients with diabetes (Nesto, "Diabetes and heart disease" in Braunwald's Heart disease, 8th Ed., p. 1547 ff.). This casts doubts on the ability of of established biomarkers to diagnose cardiac diseases and cardiac risk in this particular group of patients.

Natriuretic peptides are peptide hormones that are synthesized within the atria and ventricles in response to atrial and ventricular distension. Atrial natriuretic peptides are stored in vesicles and can readily be released after wallstress, B-type natriuretic peptide is synthesized after wall stress and appears in the circulation with some 4 to 6 hours delay. Elevated levels of B-type natriuretic peptide (BNP) or the prohormone NT-pro BNP have been linked to the extent of heart failure and heart disease mortality (Kempf et al., 2009, Circ Cardiovasc. Genet 2: 286-292). Conversely in patients presenting with chronic dyspnea NT-pro BNP was able to rule out heart failure if NT-pro BNP was below 125 pg/ml (Svendstrup Nielsen, 2004, Eur. J. of Heart Failure, 6: 63-70). A cut off value of 125 pg/ml was also suggested in patients without symptoms of heart failure but the presence of hypertension or diabetes mellitus to rule out left ventricular dysfunction as assessed by echocardiography (Betti I. et al., 2009, J Card Fail. 15: 377-384). Recently, growth differentiation factor 15 (GDF-15) has been introduced as a novel biomarker for assessing the prognosis of patients with cardiovascular diseases. In a group of patients with stable coronary artery disease the following mortality rates have been determined: GDF-15 below 1200 ng/1: 1.4 % within 3.6 years, GDF-15 between 1200 and 1800 ng/1: 2.7 % within 3.6 years and GDF-15 above 1800 ng/1: 15.0 % within 3.6 years. This renders GDF-15 as a strong indicator of heart failure in a population with chronic artery disease and also as a strong prognostic indicator for cardiac death (Kempf et al., 2009, Circ. Cardiovasc. Genet. 2: 28-292, 2009). Previous work has shown that a Troponin T test with a sensitivity of 3 pg/ml detected Troponin T concentration not only in patients with acute coronary syndrome and myocardial infarction but also in patients with clinically stable chronic artery disease. (EP 07114174.1 / 1 890 154). Using Troponin T quartiles it could be demonstrated that Troponin T concentrations correlated with NT-proBNP levels and, thus, with the extent of heart failure. In addition the level of sensitive Troponin T has been shown to be linked to future heart failure and its frequency (Omland et al., 2009, New England Journal of Medicine, 361: 2538-2547).

Previously it has been documented that patients with type 2 diabetes mellitus with NT-proBNP concentrations above 33 pg/ml (but still below 125 pg/ml) suffered more frequently from cardiovascular events as compared to a group of type 2 diabetes patients with NT-proBNP levels below 33 pg/ml. (EP 05 014 521). This suggests that NT-pro BNP amounts within the normal range apparently do not rule out cardiac disorders or cardiac risk factors in all patients suffering from diabetes mellitus.

Even more confusing, increased levels of GDF-15 close to those found in patients with cardiovascular disease and at increased risk of mortality have been reported in patients with diabetes mellitus, however in this study NT-proBNP or other cardiac markers were not determined (Dostalova et al., 2009, Europ Heart Journal 3: 2346-2353). Thus, in type 2 diabetes patients the information provided by natriuretic peptides (BNP or NT-proBNP) appears to be controversial to that of GDF-15 with NT-proBNP excluding a relevant heart disease and GDF-15 suggesting a poor cardiac prognosis. Thus there is an urgent need to resolve these seemingly controversial information and establish diagnostic tools to reliably identify or exclude cardiac disease in the highly prevalent diabetes population.

A further need in the treatment and prevention of Type 2 diabetes is the recognition of those who do not meet the criteria of type 2 diabetes mellitus but are at increased risk for the development of type 2 diabetes mellitus. It is well appreciated that low adiponectin levels as a consequence of abdominal obesity and elevated CRP levels are associated with the future development of diabetes mellitus type 2. These are clearly modifiable risk factors, either through change of behaviour and/or the use of drugs such as statins or anti-inflammatory drugs such as ACE inhibitors or angiotensin receptor antagonists. (Kolberg et al., 2009 Diabetes Care, 32: 1207-1212; Urdea J et al., 2009, Journal of Diabetes Science and Technology, 3: 748-755). Both, elevated C-reactive protein and low Adiponectin are associated with increased risk of cardiovascular disease. While current strategies focus on modifiable and non-modifiable risk factors of future type 2 diabetes mellitus there is also an urgent need to identify further unconventional risk for future type 2 diabetes.

Given the above described differences of several well known biomarkers for cardiac diseases in diabetic patients there is an urgent need for the identification of biomarkers that reliably diagnose cardiac diseases in this particular group of patients where many established biomarkers fail to yield reliable information.

The problem underlying the present invention is the provision of biomarkers and methods related to these biomarkers which permit to identify or diagnose a cardiac disorder in patients suffering from diabetes mellitus, in particular type 2 diabetes mellitus. In a preferred embodiment, the patient has amounts of a natriuretic peptide, preferably NT-proBNP, and/or GDF-15 which do not permit to diagnose the cardiac state of the patient. Preferably, the amounts of the natriuretic peptide and, preferably, GDF-15 are controversial, i.e. one of the markers is indicative for a cardiac disease and the other not.

The technical problem is solved by the embodiments characterized in the claims and herein below.

The present invention relates to a method for diagnosing a cardiac disorder in a diabetes patient having amounts of a natriuretic peptide or a variant thereof and GDF-15 or a variant thereof that provide contradictory information based on the determination of the amount of a cardiac troponin or a variant thereof and/or the amount of H-FABP or a variant thereof in a sample of the patient and the comparison of the measured amounts of the markers to reference amounts.

Preferably, the method of the present invention comprises the steps of a) determining the amount of a cardiac troponin or a variant thereof and/or H-FABP or a variant thereof in a sample of the patient; b) comparing the amounts determined in step a) to reference amounts; and c) diagnosing the cardiac disorder based on the result of the comparison of step b).

It is also provided for a method for diagnosing a cardiac disorder in a diabetes patient having amounts of a natriuretic peptide or variant thereof and GDF-15 or a variant thereof that provide contradictory information comprising the steps of
a) determining the amount of a cardiac troponin or a variant thereof and/or H-FABP or a variant thereof in a sample of the patient; and
b) comparing the amounts determined in step a) to reference amounts to diagnose the cardiac disorder.

Accordingly, the present invention relates to a method for diagnosing a cardiac disorder in a diabetes patient having amounts of a natriuretic peptide or a variant thereof and of GDF-15 or a variant thereof that provide contradictory information comprising the steps of
a) determining the amount of a cardiac troponin or a variant thereof and/or of H-FABP or a variant thereof;
b) comparing amounts determined in step a) to reference amounts; and
c) diagnosing the cardiac disorder based on the comparison of step b).

In a prefered embodiment of the invention, steps b) and c) as disclosed beforehand are carried out in one single step. In this embodiment, the method of the invention of diagnosing a cardiac disorder comprises step b) diagnosing the cardiac disorder by comparing the amounts determined in step a) to reference amounts.

The method of the present invention is, preferably, an in vitro method. Moreover, it may comprise steps in addition to those explicitly mentioned above including sample pretreatments or evaluation of the results obtained by the method. The above-described method as well as any other method of the present invention may be carried out manually and/or assisted by automation. Preferably, steps (a), (b) and/or (c) may in total or in part be assisted by automation including suitable robotic and sensory equipment for the determination in step (a), and/or a computer-implemented comparison under steps (b) and (c).

It is to be understood that the method of the present invention, preferably, also includes a step of determining the amount of a natriuretic peptide or a variant thereof and the amount of GDF-15 or a variant thereof and a further step of comparing the determined amounts of the natriuretic peptide or the variant thereof and of GDF-15 or the variant thereof to reference amounts, prior to carrying out step a) of the method of the present invention as laid out above, in order to establish whether the patient is suitable for a diagnosis according to the method of the present invention. Thus, a comprehensive assessment of the patient's condition based on a single sample is possible.

The term "cardiac disorder" refers to any cardiac disease that impairs the integrity of cardiomyocytes. This impaired integrity of cardiomyocytes leads to the release of their contents into the bloodstream. The increased amounts of troponins and H-FABP are explained by this. As is known in the art troponins may be released by cardiomyocytes undergoing necrosis. If in the individual of the invention increased amounts of GDF-15 are found (as inflammatory marker) the cardiac disease has, preferably, an inflammatory component. This is shown in the study underlying the present invention (see example 2), Most preferably, the cardiac disease is associated with fibrotic processes. Since fibrotic processes in the myocardium cause a stiffening of the myocardium, the cardiac disease is, more preferably, associated with a diastolic dysfunction. Said diastolic dysfunction does, preferably, not cause symptoms in the patient that induce him/her to seek medical assistance.

The patient in accordance with the present invention suffers from diabetes mellitus. He/she is, preferably, a human. With respect to cardiac diseases and disorders the patient is, preferably, apparently healthy. A patient is apparently healthy with respect to cardiac disorders if he/she does not show obvious symptoms of a cardiac disease or disorder and such a disease or disorder has not been diagnosed in the patient. More preferably, the patient shall not suffer from exercise induced chest pain or from dyspnea. Preferably, the blood pressure of the patient does not exceed 140 mmHg (systolic) and/or 90 mmHg (diastolic). More preferably the hypertension of the patient at maximum stage 1 hypertension, i.e. less then 160 mmHg systolic and 100 mmHg diastolic. In addition to this, the patient is, preferably, stable, i.e. his/her condition does not deteriorate and has not deteriorated rapidly in the previous six months. More preferably, the patient has not suffered from acute cardiovascular events within at least about 3 months, at least about 6 months or at least about 9 months prior to the diagnosis according to the method of the present invention. The term "acute cardiovascular event" refers to myocardial infarction with or without ST segment elevation, instable angina pectoris and stroke. Preferably, the kidney function of the patient is normal, i.e. the glomerular filtration rate is higher than 60 ml per minute and 1.73 m² body surface or by the serum creatinine value is lower than 1.3 mg/dl.

The term "amounts of a natriuretic peptide or a variant thereof and GDF-15 or a variant thereof that provide contradictory information" refers to amounts of said markers that do no allow a reliable diagnosis of a cardiac disorder because the diagnostic information given by one marker does not match with the information provided by the other marker. The said term, preferably, refers to the case that the information given by both markers is contradictory. More preferably, the measured amount of the natriuretic peptide or the variant thereof indicates that the patient is healthy with respect to cardiac disorders while the measured amount of GDF-15 or the variant thereof indicates that the patient suffers from a severe cardiac disorder. Severe cardiac disorders are preferably heart failure with or without chronic artery disease or acute coronary sydrome. Wollert et al Circulation 2007: 115: 962 - 971) have described increased mortality in patients with ACS and GDF 15 levels above 1800 pg/ml, similar results have been obtained in patients with established CHF (Kempf T. et al (J. Am Coll Cardiol 2007: 50: 1054 - 1060) where the levels of GDF 15 could also be correlated to NYHA classes.

Because in addition to this, the patient, preferably, shall be apparently healthy with respect to cardiac disorders, the high amount of GDF-15 is not plausible (and, hence, contradictory to the information provided by the amount of GDF-15 and also the fact that the individual is apparently healthy). The amount of GDF-15 that provides contradictory information is, preferably, even higher than in patients with stable coronary artery disease. Even more preferably, the amount of the natriuretic peptide or the variant thereof that provides contradictory information in the patient is lower than about 125 pg/ml, lower than about 100 pg/ml, lower than about 75 pg/ml or lower than about 50 pg/ml and the amount of GDF-15 or the variant thereof is higher than about 750 pg/ml, higher than about 1000 pg/ml, higher than about 1250 pg/ml or higher than about 1500 pg/ml. Most preferably, the amount of the natriuretic peptide or the variant thereof that provides contradictory information in the patient is lower than about 75 pg/ml and the amount of GDF-15 or the variant thereof that provides contradictory information is higher than about 1000 pg/ml. The term "about" as used herein refers to +/- 20%, more preferably +/-10%, most preferably, +/- 5% of a given measurement or value. An amount of GDF-15 higher than the reference amount and an amount of the natriuretic peptide lower than the reference amount indicate that the patient is eligible for a diagnosis according to the method of the present invention.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The term "diabetes" refers to a physiological condition which is characterized by increased amounts of glucose in the blood of the patient. There are two main types of diabetes:
In type 1 diabetes the secretion of insulin by the pancreas is impaired so that a lack of insulin causes the elevated glucose levels in the blood. Typically, type 1 diabetes is caused by an autoimmune reaction that leads to the destruction of the insulin producing beta cells of the pancreas. Control of this type of diabetes is comparatively easily achieved by insulin substitution therapy.
Type 2 diabetes is characterized by increased insulin resistance of the body. A normal or even an elevated secretion of insulin is not sufficient to induce glucose uptake into the cells of the body. Blood glucose levels are, therefore, elevated. In early type 2 diabetes insulin secretion is often increased as a compensation for the reduced effect of insulin. Later in the course of the disease, insulin secretion, typically, drops or stops completely. Type 2 diabetes is, preferably, diagnosed by an oral glucose tolerance test (OGTT), by the determination of fasting glucose levels or by determining the fraction of glycated hemoglobin.

For the determination of glucose tolerance the patient is given a glucose solution containing a defined amount of glucose (e.g. a total amount of 75 g glucose or 1.75 g glucose per kg body weight). Blood glucose levels are determined before the administration of the glucose solution and 2 hours after the administration. Blood glucose levels before the administration of glucose of more than 126 mg/dl are indicative of diabetes. Levels between 110 mg/dl and 125 mg/dl are indicative of impaired fasting glycemia. A fasting glucose level below 110 mg/dl indicates that the individual does not suffer from diabetes. A blood glucose level after 2 hours of more than 200 mg/dl is indicative of diabetes. Levels between 140 mg/dl and 200 mg/dl indicate an impaired glucose tolerance. A level below 140 mg/dl indicates a normal glucose tolerance.

The fraction of glycated hemoglobin A1c is a measure for the average blood level over a longer time. During the life span of a red blood cell which lasts approximately 120 days glucose reacts with hemoglobin and glycated hemoglobin is formed by an irreversible reaction. The more glucose is present in the blood, the more glycated hemoglobin is formed. Thus, the level of glycated hemoglobin Alc indicates the average blood glucose level during the past 20 to 120 days. HbA1c levels calculated according to International Federation of Clinical Chemistry (IFCC) of more than 53 mmol/mol are indicative of type 2 diabetes.

The term "cardiac disorder" refers to any structural or functional impairment of the myocardium. Because cardiac troponins are released from dead or dying cardiomyocytes, elevated amounts of cardiac troponins indicate necrotic processes in the myocardium. Therefore, the term "cardiac disorder", more preferably, refers to a condition of the myocardium that is associated with necrosis of cardiomyocytes. The absence of angina pectoris in the diabetic patients (see example 2) indicates that there are no stenoses of major coronary arteries. Consequently, the necrosis of cardiomyocytes is, preferably, diffuse, i.e. it does affect single cardiomyocytes or small groups of cardiomyocytes randomly spread over a larger area of the myocardium rather than defined and adhering larger regions that are supplied by one major coronary artery.

The presence of high amounts of GDF-15 in these patients indicates ongoing inflammatory processes. As inflammation is frequently associated with fibrosis, i.e. the formation of scars and the replacement of functional tissue by connective tissue, the cardiac disorder is, preferably, associated with fibrosis. On a functional level, the combination of diffuse necrosis and fibrosis causes a decreased elasticity of the myocardium. A reduced elasticity, i.e. an increased stiffness, of the myocardium leads to an impaired filling of the ventricles during diastole. Thus, with respect to cardiac function the cardiac disorder is, preferably, associated with a diastolic dysfunction. As has been shown in the study underlying the present invention (see example 2), a diastolic dysfunction was diagnosed in the those diabetics who underwent dopplerechocardiography.

H-FABP is known as a marker for stunned or hibernating myocardium, see e.g. WO 2009/150181 The terms "stunning", also described as "stunned myocardium" is well known to the skilled person. This term refers to a temporal delay in the recovery of the myocardial function following ischemia, despite the fact that blood flow has been restored. A defining feature of isolated myocardial stunning is that myocardial function remains depressed while resting myocardial perfusion is normal. Thus, there is a dissociation of the usual close relationship between the subendocardial flow and function. Stunned myocardium may also occur after demand-induced ischemia. This may occur, for example, in a patient suffering from stable CAD when exercising, so that oxygen demand in myocytes exceeds supply due to a stenotic atherosclerotic plaque, limiting flow rate in a coronary artery, for example. Ischemic events leading to stunning are, for example, short term (generally < 20 min) total occlusions of a coronary artery, which may occur, for example during balloon inflation during the STENT implantation procedure. Stunning also occurs due to medium-term (0.3-5 hours) partial occlusions of a coronary artery. In stunning, the myocardial function may be regionally depressed for some time after perfusion is restored, as can be experimentally shown by measuring left ventricular wall thickening during myocardial contraction. The delay for functional restoration following restoration of blood flow generally depends on the length and severity of the ischemic event. Normally, stunning lasts from 1 hour to up to a week. If, however, repetitive reversible ischemia develops before function normalizes, stunning can lead to a state of persistent dysfunction or chronic stunning, in which the complete myocardial function is achieved only weeks after perfusion is restored. Chronic stunning may, in turn, lead to hibernating myocardium, discussed further below, in case of reversible repetitive ischemia and depressed resting flow.

The cardiac states of stunning, along with severe ischemia and wall stress are preferably diagnosed, in accordance with the invention, if the amount of sF1T1, and/or HGF, ANP-type peptide, and/or H-FABP , or of variants of any one or more of the aforementioned, preferably as determined in steps a) to c), are increased compared with their respective reference amounts.

The term "hibernating myocardium", also referred to as "myocardial hibernation" as used in this specification, is well known to the skilled person. In general, a hibernating myocardium is a condition in which myocardial contractility, metabolism and ventricular function are reduced in order to cope with a reduced oxygen supply. It is a chronic, but in many cases reversible cardiac dysfunction that is caused by prolonged myocardial hypoperfusion and that persists generally for some time that may last up to 12 months, even if normal blood flow is restored. Thus, hibernating myocardial cells are temporarily asleep, but still viable, and can "wake up" to normal function when the blood supply is fully restored by revascularization.

A "hibernating myocardium", for the purpose of the present invention, preferably is a persistent myocardial dysfunction that occurs when myocardial perfusion is chronically reduced but sufficient to maintain the viability of the myocardial tissue (see e.g. Braunwald's Heart Disease 8th Ed. 2005 Elsevier Publishers, Chapters 48 and 54).

Thus, the cardiac state or condition of a hibernating myocardium is preferably a state, in which one or a plurality of myocardial regions show a chronically depressed contractile ability, but are still viable. It is known in the art that hibernating myocardium can cause abnormal systolic or diastolic ventricular function or both. Moreover, the skilled person knows that myocardial hibernation is generally a state of persistent ventricular dysfunction that can be reversed by revascularization. Preferably, the term "hibernating myocardium" does not include myocardial stunning, which is a transient postischemic dysfunction defined elsewhere in this specification. However, it is known the art that stunning myocardium and hibernating myocardium can coexist and that stunning myocardium may turn into hibernating myocardium, particularly in cases of repetitively stunned tissues. Reviews on myocardial hibernation are, e.g., given by Heusch et al. (Am J Physiol Heart Circ Physiol 288:984-999, 2005) and Kalra et al. (Kalra DK, Zoghbi WA: Myocardial hibernation in coronary artery disease. Curr Atheroscler Rep 2002, 4:149-155). The existence of a hibernating myocardium was first shown in subjects after bypass surgery. Angiographic studies in subjects who underwent coronary angioplasty, e.g., revealed immediate recovery of global and regional systolic, as well as diastolic, function after revascularization. It has been proposed that the formation of hibernating myocardial regions is due to chronic ischemia and is a mechanism to prevent myocardial necrosis and other ischemic symptoms. The exact molecular mechanisms underlying hibernation are still not completely understood. However, they may include impaired calcium metabolism by the sarcoplasmic reticulum and reduced sensitivity of myofibrils to calcium. Metabolically, in the hibernating myocardium maximum the rate of glucose uptake during insulin stimulation and creatine phosphate and ATP levels are not regionally altered, contrasting the depressed ATP levels in stunning.

It is noted that a "hibernating myocardium", for the purpose of the present specification, preferably relates to a chronic hibernating myocardium, and preferably does not refer to short term hibernation, which refers to reduced regional oxygen consumption and energy uptake during persistent hypoperfusion. The ability of short-term hibernation to prevent necrosis is limited by the severity and duration of ischemia, with irreversible injury developing generally after periods of more than 24 hours.

The term "natriuretic peptide" comprises Atrial Natriuretic Peptide (ANP)-type and Brain Natriuretic Peptide (BNP)-type peptides and variants thereof having the same predictive potential. Natriuretic peptides according to the present invention comprise ANP-type and BNP-type peptides and variants thereof (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). ANP and BNP have a vasodilatory effect and cause excretion of water and sodium via the urinary tract. Preferably, natriuretic peptides according to the present invention are NT-proANP, ANP, and, more preferably, NT-proBNP, BNP, and variants thereof. ANP and BNP are the active hormones and have a shorter half-life than their respective inactive counterparts, NT-proANP and NT-proBNP. BNP is metabolized in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous.

The most preferred natriuretic peptides according to the present invention are NT-proBNP or variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein.

The term "cardiac Troponin" refers to all Troponin isoforms or variants thereof expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T.

Heart-type fatty acid binding protein, herein also referred to as H-FABP, is a small cytosolic protein that functions as the principal transporter of long-chain fatty acids in the cardiomyocyte, from the cell membrane to their intracellular sites of metabolism in the mitochondria, where they enter the citric acid cycle. H-FABP is present in the myocardium and it is generally thought to be released rapidly into the circulation in response to reversible cardiac dysfunction/myocardial injury. Several studies show that H-FABP is a early biochemical marker of myocardial infarction e.g. Okamoto et al., Clin Chem Lab Med 38(3):231-8 (2000) Human heart-type cytoplasmic fatty acid-binding protein (H-FABP) for the diagnosis of acute myocardial infarction. Clinical evaluation of H-FABP in comparison with myoglobin and creatine kinase isoenzyme MB; O'Donoghue et al., Circulation, 114;550-557 (2006) Prognostic Utility of Heart-Type Fatty Acid Binding Protein in patients with acute coronary syndrome or Ruzgar et al., Heart Vessels, 21;209-314 (2006) The use of human heart-type fatty acid-binding protein as an early diagnostic marker of myocardial necrosis in patients with acute coronary syndrome, and its comparison with troponinT and its creatinine kinase-myocardial band).

The term "Growth-Differentiation Factor-15" or "GDF-15" relates to a polypeptide being a member of the transforming growth factor (TGF)-β cytokine superfamily. The terms polypeptide, peptide and protein are used interchangeable throughout this specification. GDF-15 was originally cloned as macrophage-inhibitory cytokine-1 and later also identified as placental transforming growth factor-β, placental bone morphogenetic protein, non-steroidal anti-inflammatory drug-activated gene-1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal pro-peptide, GDF-15 is secreted as a ∼28 kDa dimeric protein (Bauskin 2000, Embo J 19:2212-2220). Amino acid sequences for GDF-15 are disclosed in WO99/06445, WO00/70051, WO2005/113585 Bottner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441 or Yokoyama-Kobayashi loc cit..

The term "variant" encompasses also variants of the specific peptides of the present application. Such variants have at least the same essential biological and immunological properties as the specific cardiac Troponins, GDF-15, H-FABP and natriuretic peptides. In particular, they share the same essential biological and immunological properties if they are detectable by the same specific assays referred to in this specification, e.g., by ELISA Assays using polyclonal or monoclonal antibodies specifically recognizing the said cardiac troponins, H-FABP, GDF-15 and natriuretic peptides. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific Troponin, GDF-15, H-FABP or natriuretic peptide, preferably over the entire length of the specific peptide. Variants may be allelic variants or any other species specific homologs, paralogs, or orthologs. Moreover, the variants referred to herein include fragments of the specific cardiac Troponins or natriuretic peptides or the aforementioned types of variants as long as these fragments have the essential immunological and biological properties as referred to above. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 230:177-181), Yeo et al. (Yeo 2003, Clinica Chimica Acta 338:107-115). Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T. Preferably the biological property of H-FABP is the transport of long-chain fatty acids from the cell membrane to their intracellular sites of metabolism in the mitochondria, where they enter the citric acid cycle. Thus, the variants shall be recognizable by the aforementioned means or ligands used for determination of the amount of the cardiac troponins, H-FABP, GDF-15 or natriuretic peptides. Such fragments may be, e.g., degradation products of the peptides of the present invention. Further included are variants which differ due to posttranslational modifications such as phosphorylation or myristylation.

Determining the amount of GDF-15, H-FABP, a natriuretic peptide, preferably NT-proBNP, or the amount of a cardiac troponin, preferably troponin T, or any other peptide or polypeptide or protein referred to in this specification relates to measuring the amount or concentration, preferably semi-quantitatively or quantitatively. The terms polypeptide and protein are used interchangeable throughout this application. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal - may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on EleesysTM analyzers), CBA (an enzymatic Cobalt Binding Assay, available for example on Roche-HitachiTM analyzers), and latex agglutination assays (available for example on Roche-HitachiTM analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprise the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Nonspecific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a coloured reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex- enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (US 5,744,305).

Preferably, the amounts of a cardiac troponin, GDF-15, H-FABP and a natriuretic peptide and, as the case may be, the amounts of other peptides measured in the context of the present invention are determined in a blood sample, e.g., a serum or plasma sample, obtained from a subject as defined in the present invention. Preferably, such a determination is done by ELISA. The amounts of troponin T and NT-proBNP can be determined by the COBAS assay, Roche Diagnostics Mannheim, Germany. A preferred GDF-15 assay in the context of the present invention a sandwich immuno-assay using a COBAS analyser from HTACHI-ROCHE. The assay contains antibodies obtained from R & D systems, Minneapolis, USA and the test is adopted to the assay described by Wollert et al in Clinical Chemistry 53, 2, 284 - 291, 2007. For the determination of the amount of H-FABP the HBT ELISA Test Kit for human heart type fatty acid binding protein from HyCult Biotechnology, Uden, The Netherlands is preferred.

The term "amount" as used herein encompasses the absolute amount (e.g., of a natriuretic peptide, GDF-15, H-FABP or a cardiac troponin), the relative amount or concentration (e.g, of a natriuretic peptide, GAF-15, H-FABP or a cardiac troponin) as well as any value or parameter which correlates thereto. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., expression amounts determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide, polypeptide, protein comprised by the sample to be analyzed with an amount of a reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to references which are stored in a database by a computer program. The computer program may further evaluate the result of the comparison, i.e. automatically provide the desired assessment in a suitable output format. Based on the comparison of the amount(s) determined in step a) to reference amount(s), it is possible to diagnose a cardiac disorder in said subject. It is to be understood that amounts of H-FABP and a cardiac troponin as determined in step (a) of the methods of the presents invention are compared in step (b) to reference amounts for H-FABP and a cardiac troponin as specified elsewhere in this application.

In cases where the present invention comprises the determination of the amounts of a natriuretic peptide and/or GDF-15, these amounts are compared to reference amounts for a natriuretic peptide and GDF-15, as specified elsewhere in the application. The amounts of the beforecited markers are generally carried out before determining the amount of a cardiac troponin and of H-FABP.

In general, for determining the respective amounts/amounts or amount ratios allowing to establish the desired diagnosis in accordance with the respective embodiment of the present invention, ("threshold", "reference amount"), the amount/amount(s) or amount ratios of the respective peptide or peptides are determined in appropriate patient groups.

The term "reference amount" or with respect to a cardiac troponin or H-FABP refers, preferably, to an amount of H-FABP or the cardiac troponin representing the upper level of normal found in a patient who does not suffer from a cardiac disorder. With respect to a natriuretic peptide the term "normal amount", preferably, refers to an amount of the natriuretic peptide that is lower than the upper limit of normal in patients apparently healthy with respect to cardiac disorders. With respect to GDF-15, an "increased amount" refers to an amount that is higher than the upper level of normal in healthy patients and, preferably, even in non-diabetics with coronary artery disease. The upper limit of normal is, preferably, defined by the 50th percentile, 55th percentile, 60th percentile, 65th percentile, 70th percentile, 75th percentile, 80th percentile, 85th percentile, 90th percentile, 90th percentile or 99th percentile.

The term "reference amount", preferably, refers to the amount of a cardiac troponin or H-FABP that allows the differentiation between a patient who does and a patient who does not suffer from a cardiac disorder.

According to the diagnosis to be established, the patient group may, for example, comprise only healthy individuals, or may comprise healthy individuals and individuals suffering from the pathopysiological state which is to be determined, or may comprise only individuals suffering from the pathopysiological state which is to be determined, or may comprise individuals suffering from the various pathophysiological states to be distinguished, by the respective marker(s) using validated analytical methods. In the context of monitoring the course of a disease, determining the susceptibility of a patient to a treatment or predicting the success of a treatment the groups are formed, preferably, by patients whose disease worsens and by patients whose disease improves over the course of observation. The results which are obtained are collected and analyzed by statistical methods known to the person skilled in the art. The obtained threshold values are then established in accordance with the desired probability of suffering from the disease and linked to the particular threshold value. For example, it may be useful to choose the median value, the 60th, 70th, 80th, 90th, 95th or even the 99th percentile of the healthy and/or non-healthy patient collective, in order to establish the threshold value(s), reference value(s) or amount ratios.

Consequently, threshold amounts for a cardiac troponin and H-FABP are, preferably, derived by determining the amount of the cardiac troponin and H-FABP in individuals who suffer from a cardiac disorder or in individuals who do not suffer from a cardiac disorder.

A reference value of a diagnostic marker can be established, and the amount of the marker in a patient sample can simply be compared to the reference value. The sensitivity and specificity of a diagnostic and/or prognostic test depends on more than just the analytical "quality" of the test-they also depend on the definition of what constitutes an abnormal result. In practice, Receiver Operating Characteristic curves, or "ROC" curves, are typically calculated by plotting the value of a variable versus its relative frequency in "normal" and "disease" populations. For any particular marker of the invention, a distribution of marker amounts for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create an ROC curve. For example, results of a test on "disease" samples might be ranked according to degree (say 1=low, 2=normal, and 3=high). This ranking can be correlated to results in the "normal" population, and a ROC curve created. These methods are well known in the art. See, e.g., Hanley et al, Radiology 143: 29-36 (1982).

In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement.

For NT-proBNP the reference amount is, preferably, about 90 pg/ml, about 100 pg/ml, about 110 pg/ml, about 120 pg/ml, about 130 pg/ml or about 140 pg/ml. More preferably, the reference amount is about 125 pg/ml. A patient with a lower amount of NT-proBNP than the reference amount is considered to be apparently healthy and is considered eligible for diagnosis of a cardiac disorder according to the method of the present invention.

For GDF-15 the reference amount is, preferably, about 800 pg/ml, about 1000 pg/ml, about 1200 pg/ml or about 1400 pg/ml. More preferably, the reference amount is about 1000 pg/ml. A patient with an amount of GDF-15 higher than the reference amount is considered to have an "increased amount of GDF-15" and is considered eligible for diagnosis of a cardiac disorder according to the method of the present invention.

The reference amounts of NT-proBNP and GDF-15, thus, indicates whether a patient is suitable for a diagnosis according to the method of the present invention.

With respect to troponin T the reference amount is, preferably, about 2.0 pg/ml, about 2.5 pg/ml, about 3.0 pg/ml, about 3.5 pg/ml or about 4.0 pg/ml. More preferably, the reference amount of troponin T corresponds to the amount found in the 95^{th} percentile of individuals healthy with respect to diabetes and cardiac disorders. Thus, the reference amount of troponin T is, most preferably, about 3.0 pg/ml. The term "about" as used herein refers to +/- 20%, more preferably +/-10%, most preferably, +/- 5% of a given measurement or value. A measured amount of troponin T higher than the reference amount is indicative of a cardiac disorder in the patient in question.

With respect to H-FABP the reference amount is, preferably, about 1800 pg/ml, about 2100 pg/ml, about 2400 pg/ml, about 2700 pg/ml or about 3000 pg/ml. More preferably, the reference amount of H-FABP corresponds to the amount found in the 95^{th} percentile of diabetics with a cardiac disorder. Thus, the reference amount of H-FABP is, most preferably, about 2400 pg/ml. The term "about" as used herein refers to +/- 20%, more preferably +/-10%, most preferably, +/- 5% of a given measurement or value. A measured amount of H-FABP higher than the reference amount is indicative of a cardiac disorder in the patient in question.

In an especially preferred embodiment of the present invention the absolute reference amounts for a cardiac troponin or a variant thereof and H-FABP or a variant thereof are replaced by the ratio of the amount of a cardiac troponin or a variant thereof to the amount of a natriuretic peptide or a variant thereof and the ratio of the amount of H-FABP or a variant thereof to the amount of a natriuretic peptide or a variant thereof.

Consequently, the present invention also relates to a method for diagnosing a cardiac disorder in a diabetes patient having amounts of a natriuretic peptide or variant thereof and GDF-15 or a variant thereof that provide contradictory information, comprising the steps of
a) determining the amount of a natriuretic peptide or a variant thereof and the amount of at least one marker selected from the group consisting of
   i. a cardiac troponin or a variant thereof and
   ii. H-FABP or a variant thereof;
b) calculating the ratio of the amount of the cardiac troponin or the variant thereof to the amount of the natriuretic peptide or the variant thereof and/or the ratio of the amount of H-FABP or the variant thereof to the amount of the natriuretic peptide or the variant thereof; and
c) diagnosing the cardiac disorder.

In a preferred embodiment, the cardiac disorder is diagnosed by comparing the ratios calculated in step b) to reference values.

If reference is made to ratios rather than absolute amounts of the markers of the present invention, the term "reference value" replaces the term "reference amount". The different behaviour of cardiac troponins and H-FABP on the one hand and natriuretic peptides on the other hand in diabetics is better reflected by the abovementioned ratios than by the absolute amounts of the markers of the present invention.

With respect to troponin T/NT-proBNP the reference value is about 0.20, about 0.25 or about 0.30. More preferably, the ratio is about 0.30. The term "about" as used herein refers to +/- 20%, more preferably +/-10%, most preferably, +/- 5% of a given measurement or value. A ratio of troponin T to NT-proBNP higher than said reference value is indicative of a cardiac disorder in the patient.

With respect to H-FABP/NT-proBNP the reference value is about 55, about 65, about 75 or about 86. The reference value is, more preferably, about 68 or 56 and, most preferably, 68. The term "about" as used herein refers to +/- 20%, more preferably +/-10%, most preferably, +/- 5% of a given measurement or value. A ratio of H-FABP to NT-proBNP higher than said reference value is indicative of a cardiac disorder in the patient.

The presence of a cardiac disorder in the patient has therapeutic implications. Preferably, the treatment of the diabetes of the patient is monitored more closely to ensure adequate control of blood glucose levels. In addition to this, the patient may be encouraged to modify his/her life style. This includes, preferably, weight reduction and increased exercise.

Moreover, the diagnosis of a cardiac disorder according to the method of the present invention leads, preferably, to a closer examination of the patient. For the examination of the cardiovascular system imaging methods are preferred. Preferred imaging methods are echocardiography, angiography, computed tomography with and without contrast agent and magnetic resonance tomography. Moreover, a angiography may be performed. Because the presence of a cardiac disorder indicates complications of the diabetes, the patient should be tested for non-cardiac complications of diabetes as well. Non-cardiac complications of diabetes are, preferably, diabetic nephropathy, diabetic neuropathy, diabetic retinopathy and peripheral artery disease.

Additionally, it is preferred to monitor the abovementioned parameters in short intervals to detect the onset or aggravation of any complications of diabetes early.

The management of blood glucose levels, preferably, takes into account the possible presence of microangiopathy in the patient. Thus, it therapy should not aim at blood glucose levels at the lower end of normal. Periods of hypoglycemia have to be especially avoided.

Moreover, the present invention relates to a method for deciding about further diagnostic measures and/or treatment of a cardiac disorder in a diabetes patient having amounts of a natriuretic peptide or variant thereof and GDF-15 or a variant thereof that provide contradictory information comprising the steps of
a) determining the amount of a cardiac troponin or a variant thereof and/or H-FABP or a variant thereof in a sample of the patient; and
b) comparing the amounts determined in step a) to reference amounts to decide about the further diagnostic measures and/or treatment.

Accordingly, the present invention relates to a method for deciding about further diagnostic measures and/or treatment in a diabetes patient having amounts of a natriuretic peptide or variant thereof and GDF-15 or a variant thereof that provide contradictory information comprising the steps of
a) determining the amount of a cardiac troponin or a variant thereof and/or of H-FABP or a variant thereof;
b) comparing amounts determined in step a) to reference amounts; and
c) deciding about further diagnostic measures and/or treatment based on the comparison of step b).

Step b) as laid out above, in an embodiment of the invention, comprises diagnosing a cardiac disorder by comparing the amounts of the markers as determined. The decision according to the above method is hereby established based on the diagnosis of the cardiac disorder.

Further diagnostic measures are explained above. Preferred diagnostic measures are echocardiography, angiography, computed tomography with and without contrast agent and magnetic resonance tomography as well as testing for non-cardiac complications of diabetes.

Furthermore, anti-inflammatory medication may be administered to the patient diagnosed with a cardiac disorder. Preferred anti-inflammatory medications are angiotensin receptor antagonists and ACE inhibitors.

Advantageously, the method of the present invention allows the identification of diabetics with cardiac disorders. A diagnosis based on natriuretic peptides and/or GDF-15 in this group of patients would give inconclusive results. Since the levels of natriuretic peptides in these patients are in the normal range, a diagnosis based on these markers would miss the cardiac disorder, thus preventing a timely therapy. The GDF-15 levels in these patients are high and indicate, in principle, the presence of a cardiac disorder. However, it is unclear whether the high amounts of GDF-15 in these patients are related to a cardiac disease.

Moreover, the highly elevated GDF-15 levels contradict the normal levels of natriuretic peptides. Surprisingly, the study underlying the present invention identified cardiac troponins as well as H-FABP as suitable biomarkers for cardiac disorders in diabetics. Given the fact that patients with elevated levels of cardiac troponins are expected to also show elevated levels of natriuretic peptides (see introduction), it was surprising to find out that troponin T in patients suffering from type 2 diabetes was elevated while NT-proBNP remained in the normal range. As GDF-15 and NT-proBNP in type 2 diabetics do not yield coherent results, the usefulness of H-FABP was surprising as well. The results of the study underlying the present invention indicate that the cardiac disorder in the patients causes almost no symptoms except for a diastolic dysfunction. Thus, the method of the present invention identifies diabetes patients with only mild cardiac disorders, thus allowing the early initiation of treatment of said disorder. The early initiation of treatment promises to prevent a deterioration of the cardiac disorder so that the onset of clinical symptoms can be prevented or at least delayed. By identifying those diabetes patients with cardiac disorders, diagnostic and therapeutic efforts can be focussed on the patient in special need thereof. This spares diabetics without cardiac disorders the hassles and side effects of unnecessary diagnostic and/or therapeutic procedures and at the same time allows a more economical use of the resources of the health system. As described previously NT-pro BNP levels below 125 pg/ml are believed to exclude heart failure whereas GDF 15 levels above 1200 and more preferably 1800 pg/ml indicate heart failure and severe heart failure respectively and thus do not result in a final diagnosis, this discrepancy is however resolved by the determination of troponin and/or H FABP which in case the biomarkers are increased points to an underlying cardiac disorder.

Moreover, the present invention relates to a method for monitoring the therapy of a cardiac disorder with anti-inflammatory drugs in a diabetes patient having amounts of a natriuretic peptide or variant thereof and GDF-15 or a variant thereof that provide contradictory information based on the determination of the amount of a cardiac troponin and/or H-FABP in a sample of a patient and the comparison of the measured amounts to reference amounts.

Preferably, the method of the present invention comprises the steps of a) determining the amount of a cardiac troponin or a variant thereof and/or H-FABP or a variant thereof; b) repeating step a) in at least one further sample of the patient; c) comparing the amounts determined in the first and the at least one further sample; and d) deciding based on the comparison of step c) whether the treatment of the cardiac disorder was successful.

Accordingly, the present invention relates to a method for monitoring the therapy of a cardiac disorder with anti-inflammatory drugs in a diabetes patient having amounts of a natriuretic peptide or variant thereof and GDF-15 or a variant thereof that provide contradictory information comprising the steps of
a) determining the amount of a cardiac troponin or a variant thereof and/or H-FABP or a variant thereof;
b) repeating step a) in at least one further sample of the patient;
c) comparing the amounts determined in the first and the at least one further sample; and
d) assessing based on the comparison of step c) whether the treatment of the cardiac disorder was successful.

It is also provided for a method for monitoring the therapy of a cardiac disorder with anti-inflammatory drugs in a diabetes patient having amounts of a natriuretic peptide or variant thereof and GDF-15 or a variant thereof that provide contradictory information comprising the steps of
a) determining the amount of a cardiac troponin or a variant thereof and/or H-FABP or a variant thereof;
b) repeating step a) in at least one further sample of the patient; and
c) to decide, based on the comparison, of step c) assessing whether the treatment of the cardiac disorder was successful, based on comparing the amounts determined in the first and the at least one further sample.

Anti-inflammatory drugs are, preferably, angiotensin receptor antagonists and ACE-inhibitors.

Angiotensin-receptor antagonists bind to the angiotensin-II receptor and inhibit its activity. They, thus, prevent the contraction of the smooth muscles in the walls of the blood vessels and the concomitant increase of the blood pressure. In addition to the vasodilatative effect, angiotensin receptor antagonists have anti-inflammatory effects. Preferred angiotensin receptor antagonists are Irbesartan, Telmisartan, Valsartan, Losartan and Olmesartan. Most preferred is Irbesartan.

Inhibitors of angiotensin-converting enzyme (ACE) interact at a different point with the regulation of blood pressure via angiotensin. They inhibit the action of ACE which cleaves the inactive peptide angiotensin-I to form active angiotensin-II. Similar to the angiotensin receptor antagonists ACE-inhibitors have anti-inflammatory effects. Preferred ACE-inhibitors are Captopril, Enalapril, Ramipril and Lisinopril.

Antiinflammatory effects of ACE-inhibitors and angiotensin receptor antagonists have been shown by Hoogwerf, B.J. 2010, Am. J. Cardiol., 105: 30 -35A).

In addition to the aforementioned therapies, a control of blood hypertension is desirable (NEJM March 14, 2010, ahead of print). Blood pressure is, preferably, controlled to detect increases of blood pressure even before the development of hypertension. Consequently, a treatment of increased blood pressure is, preferably, initiated before the systolic blood pressure reaches 140 mmHg and/or the diastolic blood pressure reaches 90 mmHg.

Preferably, decreasing amounts of the abovementioned biomarkers indicate, that the therapy with anti-inflammatory drugs is successful. Whether a decrease is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

A preferred embodiment of the present invention relates to a method for monitoring the treatment of a cardiac disorder with anti-inflammatory drugs in a diabetes patient having amounts of a natriuretic peptide or variant thereof and GDF-15 or a variant thereof that provide contradictory information comprising the steps of
a) determining the amount of a natriuretic peptide or a variant thereof and the amount of at least one marker selected from the group consisting of
   i. H-FABP or a variant thereof
   ii. a cardiac troponin or a variant thereof;
b) calculating the ratio of the amount of the cardiac troponin or the variant thereof to the amount of the natriuretic peptide or the variant thereof and/or the ratio of the amount of H-FABP or the variant thereof to the amount of the natriuretic peptide or the variant thereof;
c) repeating steps a) and b) in at least one further sample of the patient; and
d) assessing whether the treatment of the cardiac disorder was successful, based on comparing the ratios calculated for the first and the at least one further sample..

Moreover, the present invention relates to a device for diagnosing a cardiac disorder in a diabetes patient having amounts of a natriuretic peptide or variant thereof and GDF-15 or a variant thereof that provide contradictory information comprising
a) an analyzing unit for determining the amount of a cardiac troponin or a variant thereof and/or the amount of H-FABP or a variant thereof in a sample of the patient; and
b) an evaluation unit for comparing the measured amounts of the markers to reference amounts.

In a preferred embodiment of the present invention the analyzing unit further comprises means for determining the amount of a natriuretic peptide or a variant thereof in a sample of the patient and the evaluation unit further comprises means for calculating the ratio of the amount of the cardiac troponin to the amount of the natriuretic peptide and the ratio of the amount of H-FABP to the amount of the natriuretic peptide.

The term "device" as used herein relates to a system of means comprising at least the aforementioned means operatively linked to each other as to practise the method of the present invention. Preferred means for determining the amounts of the markers of the present invention, and means for carrying out the comparison are disclosed above in connection with the method of the invention. How to link the means in an operating manner will depend on the type of means included into the device. For example, where an analysis unit for automatically determining the amount of the gene products of the present invention is applied, the data obtained by said automatically operating analysis unit can be processed by, e.g., a computer as evaluation unit in order to obtain the desired results. Preferably, the means are comprised by a single device in such a case.

Said device, preferably, includes an analyzing unit for the measurement of the amounts of the peptides of the present invention in an applied sample and an evaluation unit for processing the resulting data. Preferably, the evaluation unit comprises a database with the stored reference amounts and a computer program code which when tangibly embedded on a computer carries out the comparison of the determined amounts and the reference amounts stored in the database. For the comparison of the amounts of the markers of the present invention determined in a first and at least second further sample it is preferred that the evaluation unit comprises storage space for storing the determined amounts. More preferably, the evaluation unit comprises a further computer program code which allocates the result of the comparison to a risk prediction or diagnosis.

Alternatively, where means such as test stripes are used for determining the amounts of the peptides of the present invention, the evaluation unit may comprise control stripes or tables allocating the determined amount to a reference amount. The test stripes are, preferably, coupled to a ligand which specifically binds to the peptides of the present invention. The strip or device, preferably, comprises means for detection of the binding of the peptides of the present invention to the said ligand. Preferred means for detection are disclosed in connection with embodiments relating to the method of the invention above. In such a case, the analysis unit and the evaluation unit are operatively linked in that the user of the system brings together the result of the determination of the amount and the diagnostic or prognostic value thereof due to the instructions and interpretations given in a manual. The analysis unit and the evaluation unit may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the gene product, Plasmon surface resonance devices, NMR spectrometers, mass-spectrometers etc.) or evaluation units/devices referred to above in accordance with the method of the invention.

Moreover, the present invention relates to a kit for diagnosing a cardiac disorder in a diabetes patient having amounts of a natriuretic peptide or variant thereof and GDF-15 or a variant thereof that provide contradictory information comprising
a) an analyzing agent for determining the amount of a cardiac troponin or a variant thereof and/or the amount of H-FABP or a variant thereof in a sample of the patient; and
b) an evaluation unit for comparing the measured amounts of the markers to reference amounts.

In a preferred embodiment of the present invention the kit further comprises an analyzing agent for determining the amount of a natriuretic peptide or a variant thereof in a sample of the patient and the evaluation unit further comprises means for calculating the ratio of the amount of the cardiac troponin to the amount of the natriuretic peptide and the ratio of the amount of H-FABP to the amount of the natriuretic peptide.

The term "kit" as used herein refers to a collection of the aforementioned components of which may or may not be packaged together. The components of the kit may be comprised by separate vials (i.e. as a kit of separate parts) or provided in a single vial. Moreover, it is to be understood that the kit of the present invention is to be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention. The kit shall comprise an analyzing agent. This agent is capable of specifically recognizing the peptides of the present invention in a sample of the subject. Moreover, the said agent shall upon binding to the peptides of the present invention, preferably, be capable of generating a detectable signal, the intensity of which correlates to the amount of the peptides of the present invention present in the sample. Dependent on the type of signal which is generated, methods for detection of the signal can be applied which are well known in the art. Analyzing agents which are preferably used for the kit of the present invention include antibodies or aptamers. The analyzing agent may be present on a test stripe as described elsewhere herein. The amounts of of the peptides of the present invention thus detected can then be further evaluated in the evaluation unit. Preferred evaluation units to be used for the kit of the present invention include those referred to elsewhere herein.

The present invention also relates to the use of a kit or device for determining the amount of a cardiac troponin or a variant thereof and/or the amount of H-FABP or a variant thereof in a sample of a subject, comprising means for determining the amount of the cardiac troponin or the variant thereof and/or the amount of H-FABP or the variant thereof and/or means for comparing the amount of the cardiac troponin or the variant thereof and/or of H-FABP or the variant thereof to at least one reference amount for: diagnosing a cardiac disorder in a diabetes patient having amounts of a natriuretic peptide or variant thereof and GDF-15 or a variant thereof that provide contradictory information.

The present invention also relates to the use of: an antibody against a cardiac troponin or a variant thereof and/or an antibody against H-FABP or a variant thereof and/or of means for determining the amounts of a cardiac troponin or a variant thereof and/or the amount of H-FABP or a variant thereof and/or of means for comparing the amount of the cardiac troponin or the variant thereof and/or the amount of H-FABP or the variant thereof to at least one reference amount for the manufacture of a diagnostic composition for: diagnosing a cardiac disorder in a diabetes patient having amounts of a natriuretic peptide or variant thereof and GDF-15 or a variant thereof that provide contradictory information.

The present invention also relates to the use of: an antibody against a cardiac troponin or a variant thereof and/or an antibody against H-FABP or a variant thereof and/or of means for determining the amounts of a cardiac troponin or a variant thereof and/or of H-FABP or a variant thereof and/or of means for comparing the amounts of the cardiac troponin or the variant thereof and/or the amount of H-FABP or the variant thereof to at least one reference amount for: diagnosing a cardiac disorder in a diabetes patient having amounts of a natriuretic peptide or variant thereof and GDF-15 or a variant thereof that provide contradictory information.

Moreover, the present invention relates to a method for identifying an individual with an increased risk of suffering from type 2 diabetes in the future based on the determination of the amount of a cardiac troponin or a variant thereof in a sample of the individual and the comparison of the determined amount of the cardiac troponin or the variant thereof to a reference amount.

Preferably, the method of the present invention comprises the steps of a) determining the amount of a cardiac troponin or a variant thereof in a sample of the individual; b) comparing the amount of the cardiac troponin or the variant thereof determined in step a) to a reference amount; and c) deciding whether the individual has an increased risk of suffering from type 2 diabetes in the future based on the result of the comparison in step b).

Consequently, the present invention relates to a method for identifying an individual with an increased risk of suffering from type 2 diabetes in the future comprising the steps of
a) determining the amount of a cardiac troponin or a variant thereof in a sample of the individual; and
b) comparing the amount of the cardiac troponin or the variant thereof determined in step a) to a reference amount to decide whether the individual has an increased risk of suffering from type 2 diabetes in the future.

Also preferably, the method for identifying an individual with an increased risk of suffering from type 2 diabetes in the future comprises the steps of
a) determining the amount of a cardiac troponin or a variant thereof in a sample of the individual;
b) comparing the amount of the cardiac troponin or the variant thereof determined in step a) to a reference amount; and
c) deciding whether the individual has an increased risk of suffering from type 2 diabetes in the future based on the results of the comparison in step b).

The individual is, preferably, a human. The individual is, preferably, apparently healthy with respect to type 2 diabetes and cardiac diseases that are known to increase the amount of cardiac troponins in the blood. It is known that the amounts of cardiac troponins are increased in a large number of cardiac diseases, and these diseases are known to the person skilled in the art.

An individual is apparently healthy with respect to cardiac diseases if it does not show obvious symptoms of a cardiac disease or disorder and such a disease or disorder has not been diagnosed in the individual. Non-limiting examples include the cardiac diseases disclosed above, in particular coronary artery disease CAD, dyspnea, chest pain, preferably exercise induced chest pain. Furthermore, the individual shall not have a history of acute cardiovascular events, as specified below. Coronary artery disease (CAD), also called atherosclerotic heart disease, is well known in the art and is generally the consequence of the accumulation of atheromatous plaques within walls of the coronary arteries that supply the myocardium with oxygen and nutrients. CAD may progress over many years and may result in significant stenoses of coronary arteries, severely restricting the flow of oxygen-carrying blood to the myocardium. Individuals suffering from CAD may suffer from unstable angina pectoris UAP. Individuals suffering from CAD frequently show increased amounts of a cardiac troponin, due to necrotic events to the cardiomyocytes as a consequence of ischemia caused by the atheromatous plaques. CAD may lead to myocardial ischemia and myocardial infarction, for example.

An individual is apparently healthy with respect to diabetes if it does not show obvious signs and symptoms of the disease. Preferably, the fasting glucose and the glucose tolerance as determined with an OGTT are not in the diabetic range and the patient does not show evidence of hyperinsulinemia. More preferably, the fasting glucose is not impaired, i.e. the fasting glucose level is below 110 mg/dl, and the OGTT does not indicate a decreased glucose tolerance, i.e. the blood glucose level 2 hours after the oral administration of glucose is below 140 mg/dl.

As long as the above mentioned criteria are fulfilled, the individual may, however, show known risk factors of future type 2 diabetes. These risk factors are, preferably, adipositas, a sedentary life style with lack of exercise and increased levels of vitamin D.

The individual is, preferably, stable, i.e. its condition does not deteriorate and has not deteriorated rapidly in the previous six months. More preferably, the individual has not suffered from acute cardiovascular events within at least about 3 months, at least about 6 months or at least about 9 months prior to the diagnosis according to the method of the present invention. The term "acute cardiovascular event" refers to myocardial infarction with or without ST segment elevation, instable angina pectoris and stroke.

Preferably, the kidney function of the individual is normal, i.e. the glomerular filtration rate is higher than 60 ml per minute and 1.73 m² body surface or by the serum creatinine value is lower than 1.3 mg/dl.

The reference amount of troponin T that is indicative of an increased risk of an individual to suffer from type 2 diabetes in the future is, preferably, about 2.5 pg/ml, about 3.0 pg/ml or about 3.5 pg/ml. Most preferably the reference amount is about 3.0 pg/ml. The term "about" as used herein refers to +/- 20%, more preferably +/-10%, most preferably, +/- 5% of a given measurement or value. A measured amount of troponin T higher than the reference amount is indicative of an increased risk of the individual to suffer from type 2 diabetes in the future.

The term "deciding whether an individual has an increased risk" used herein refers to determining the probability according to which a subject will suffer from a disease or condition referred to herein within a defined time window (predictive window) in the future. The predictive window is an interval in which the subject shall develop the disease or condition according to the predicted probability. The predictive window may be the entire remaining lifespan of the subject upon analysis by the method of the present invention. Preferably, however, the predictive window is an interval of about 1 year, about 3 years, about 6 years, about 9 years, about 12 years, about 15 years or about 18 years after the sample to be analyzed by the method of the present invention has been obtained. more preferably, the predictive window is about 12 years. The term "about" as used herein refers to +/- 20%, more preferably +/-10%, most preferably, +/- 5% of a given measurement or value. As will be understood by those skilled in the art, such an assessment is usually not intended to be correct for 100% of the subjects to be analyzed. The term, however, requires that the assessment will be valid for a statistically significant portion of the subjects to be analyzed. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

Preferably, the term "increased risk" according to the present patent application refers to an absolute risk of at least about 11 %, at least about 13 % at least about 15 % or at least about 17 % to suffer from type 2 diabetes within the predictive window. More preferably an increased risk is an absolute risk of at least about 13 %. The term "about" as used herein refers to +/- 20%, more preferably +/-10%, most preferably, +/- 5% of a given measurement or value.

In relative terms, an increased risk is a risk that is increased by at least about 75 %, at least about 100 % or at least about 125 % as compared to the risk of a reference group of individuals. Said reference group, preferably, consists of individuals with amounts of troponin T of less than about 3 pg/ml.The term "about" as used herein refers to +/- 20%, more preferably +/-10%, most preferably, +/- 5% of a given measurement or value.

In a preferred embodiment of the present invention the amount of GDF-15 or a variant thereof is determined in step a) of the method of the present invention and the determined amount of GDF-15 or the variant thereof is compared to a reference amount in step b) to decide whether the individual has an increased risk of suffering from type 2 diabetes in the future.

For GDF-15 alone, the reference amount is preferably, about 550 pg/ml, about 650 pg/ml, about 750 pg/ml or about 850 pg/ml. More preferably, the reference amount corresponds to the 50th or 75th percentile in a population of apparently healthy individuals. Thus, the reference amount for GDF-15 is, most preferably, about 565 pg/ml or about 775 pg/ml. A measured amount of GDF-15 higher than the reference amount is indicative of an increased risk of the individual to suffer from type 2 diabetes within the predictive window. The term "about" as used herein refers to +/- 20%, more preferably +/-10%, most preferably, +/- 5% of a given measurement or value.

Based on the determination of troponin T and GDF-15 an "increased risk" is a absolute risk of suffering from type 2 diabetes within the predictive window is, preferably, at least about 12 %, at least about 13 %, at least about 14 %, at least about 15 %, at least about 16 %, at least about 17 % or at least about 18 % if the amount of troponin T is above 3 pg/ml and the amount of GDF-15 is above the median. More preferably, the absolute risk is at least about 15.3 %. The term "about" as used herein refers to +/- 20%, more preferably +/-10%, most preferably, +/- 5% of a given measurement or value.

An individual with a low risk of suffering from type 2 diabetes within the predictive window is, preferably, an individual with troponin amount below about 3 pg/ml and GDF-15 below the 50^{th} percentile. More preferably, the individual with a low risk has an amount of troponin T below 3 pg/ml and an amount of GDF-15 below 564 pg/ml. Said individual has an absolute risk of suffering from type 2 diabetes of at least about 2 %, at least about 3 %, at least about 4 %, at least about 5 % or at least about 6 %. More preferably, a low risk is a risk of about 4.2 %. The term "about" as used herein refers to +/- 20%, more preferably +/-10%, most preferably, +/- 5% of a given measurement or value.

In relative terms the risk of an individual with a an increased risk is increased by at least about 100 %, at least about 200 %, at least about 300 %, at least about 400 % or at least about 500 % as compared to an individual with a low risk. More preferably, the risk of suffering from type 2 diabetes within the predictive window is increased by at least about 290 %. The term "about" as used herein refers to +/- 20%, more preferably +/-10%, most preferably, +/- 5% of a given measurement or value.

Thus, the present invention not only allows the decision whether an individual has an increased risk of suffering from type 2 diabetes in the future, it also allows the quantification of said risk in absolute as well as in relative terms.

Consequently, the present invention also relates to a method for predicting the risk of an individual to suffer from type 2 diabetes in the future comprising the steps of
a) determining the amount of a cardiac troponin or a variant thereof in a sample of the individual;
b) comparing the amount of the cardiac troponin or the variant thereof determined in step a) to a reference amount; and
c) calculating the individual's risk of suffering from type 2 diabetes in the future based on the results of the comparison in step b).

The risk of suffering from type 2 diabetes in the future is, preferably, the absolute or relative risk as defined above.

A patient with an increased risk of suffering from type 2 diabetes in the future is, preferably, closely monitored for the first signs of increased insulin resistance or even diabetes. Thus, the disease can be detected and treated early so that the probability of the devastating consequences of an untreated diabetes such as diabetic nephropathy, diabetic cardiomyopathy, diabetic retinopathy, coronary artery disease and peripheral vascular disease are prevented or at least delayed. Moreover, preventive measures can be taken to prevent or delay the onset of the diabetes itself. Preventive measures are preferably lifestyle modification, in particular weight reduction and increased exercise, and medication with metformin, rosiglitazone or valsartan. Additionally the anticoagulation therapy, preferably with acetylsalicylic acid, is a treatment option.

It has been found in the study underlying the present invention that cardiac troponins and GDF-15 can predict the future onset of type 2 diabetes mellitus in apparently healthy patients. Cardiac troponins are well known markers for myocardial necrosis and have not been implicated in the pathophysiology of type 2 diabetes. Since diabetes is a metabolic disorder and myocardial necrosis may be a consequence of complications of diabetes, it was surprising that a marker for structural myocardial damage can predict the onset of a metabolic disorder.

As adiponectin is the only other known biomarker for determining the risk of future type 2 diabetes, a further preferred embodiment of the present invention relates to the determination of the amount of adiponectin a sample of the patient in addition to the amounts of a cardiac troponin or a variant thereof and/or GDF-15 or a variant thereof and its comparison to a reference amount.

If the measured amount of adiponectin is lower than the reference amount, i.e. the lower limit of normal in individuals without type 2 diabetes or the metabolic syndrome, weight reduction and/or the administration ACE-inhibitors and/or the administration of statins is recommended.

In a preferred embodiment of the present invention the individual whose risk of developing type 2 diabetes is to be diagnosed is a patient who suffers from the metabolic syndrome. Because the metabolic syndrome may be associated with high blood pressure, the patient may suffer from arterial hypertension. However, the patient does, preferably, not suffer from a symptomatic cardiac disease. More preferably, the patient does not suffer from exercise induced chest pain or dyspnea or from coronary artery disease. For the diagnosis of the metabolic syndrome the EGIR criteria (European Group for the Study of Insulin Resistance) are, preferably, applied. According to the EGIR criteria, a patient suffers from the metabolic syndrome if he/she has serum insulin levels larger than 44 pmol/l and additionally meets at least two of the following criteria: (i) BMI above 30 kg/m², (ii) S-triglyceride levels above 2 mmol/l or HDL cholesterol below 1 mmol/l, (iii) fasting glucose above 6.1 mmol/l and (iv) systolic blood pressure above 140 mmHg and/or diastolic blood pressure above 90 mmHg.

In patients with the metabolic syndrome the determination of GDF-15 or a variant thereof is preferred.

In a patient with the metabolic syndrome, the reference amount of GDF-15 is preferably, about 450 pg/ml, about 500 pg/ml, about 550 pg/ml or about 600 pg/ml. More preferably, the reference amount corresponds to the 25th percentile in a population of patients suffering from the metabolic syndrome. Thus, the reference amount for GDF-15 is, most preferably, about 490 pg/ml. A measured amount of GDF-15 higher than the reference amount is indicative of an increased risk of the patient to suffer from type 2 diabetes within the predictive window. The term "about" as used herein refers to +/- 20%, more preferably +/-10%, most preferably, +/- 5% of a given measurement or value.

Based on the determination of GDF-15 in a sample from a patient suffering from the metabolic syndrome an "increased risk" is an absolute risk of suffering from type 2 diabetes within the predictive window of, preferably, at least about 20 %, at least about 24 %, at least about 28 %, at least about 32 % or at least about 36 % if the amount of GDF-15 is higher than the reference amount. More preferably, the absolute risk is at least about 28 %. The term "about" as used herein refers to +/- 20%, more preferably +/-10%, most preferably, +/- 5% of a given measurement or value.

In relative terms, the risk is preferably increased by at least 50 %, at least about 75 %, at least about 100 %, at least about 150 % or at least about 200 % as compared to a patient with low risk. The patient with a low risk of developing type 2 diabetes within the predictive window is, preferably, a patient with an amount of GDF-15 in the lowest quartile. The term "about" as used herein refers to +/- 20%, more preferably +/-10%, most preferably, +/- 5% of a given measurement or value.

Moreover, the present invention relates to a device for identifying an individual with an increased risk of suffering in the future from type 2 diabetes comprising
a) an analyzing unit for determining the amount of a cardiac troponin or a variant thereof in a sample of the patient; and
b) an evaluation unit for comparing the measured amount of the cardiac troponin or the variant thereof to a reference amount.

In a preferred embodiment of the present invention the analyzing unit further comprises means for determining the amount of GDF-15 or a variant thereof in a sample of the patient and the analyzing unit further comprises means for comparing the determined amount of GDF-15 or the variant thereof to a reference amount.

Moreover, the present invention relates to a kit for identifying an individual with an increased risk of suffering in the future from type 2 diabetes comprising
a) an analyzing agent for determining the amount of a cardiac troponin or a variant thereof in a sample of the patient; and
b) an evaluation unit for comparing the measured amount of the cardiac troponin or the variant thereof to a reference amount.

In a preferred embodiment of the present invention the kit further comprises an analyzing agent for determining the amount of GDF-15 or a variant thereof in a sample of the patient and the analyzing unit further comprises means for comparing the determined amount of GDF-15 or the variant thereof to a reference amount.

The present invention also relates to the use of a kit or device for determining the amount of a cardiac troponin or a variant thereof and optionally the amount of GDF-15 or a variant thereof in a sample of a subject, comprising means for determining the amount of the cardiac troponin or the variant thereof and optionally the amount of GDF-15 or the variant thereof and/or means for comparing the amount of the cardiac troponin or the variant thereof and optionally the amount of GDF-15 or the variant thereof to at least one reference amount for: identifying an individual with an increased risk of suffering in the future from type 2 diabetes.

The present invention also relates to the use of: an antibody against a cardiac troponin or a variant thereof and optionally the amount of GDF-15 or a variant thereof and/or of means for determining the amounts of a cardiac troponin or a variant thereof and optionally the amount of GDF-15 or the variant thereof and/or of means for comparing the amount of the cardiac troponin or the variant thereof and optionally the amount of GDF-15 or the variant thereof to at least one reference amount for the manufacture of a diagnostic composition for: identifying an individual with an increased risk of suffering in the future from type 2 diabetes.

The present invention also relates to the use of: an antibody against a cardiac troponin or a variant thereof and optionally the amount of GDF-15 or a variant thereof and/or of means for determining the amounts of a cardiac troponin or a variant thereof and optionally the amount of GDF-15 or the variant thereof and/or of means for comparing the amounts of the cardiac troponin or the variant thereof and optionally the amount of GDF-15 or variant thereof to at least one reference amount for: identifying an individual with an increased risk of suffering in the future from type 2 diabetes.

The identification of patients with an increased risk of suffering from type 2 diabetes allows the concentration of preventive efforts on those patients with the greatest need. In the case of lifestyle modification this may be of lesser importance since a healthy lifestyle is not harmful for the individual. However, medication for the prevention of diabetes is costly and may have undesired side effects. Thus, it should only to individuals where the benefit, i.e. the prevention or delayed onset of diabetes, outweigh the costs and risks. The targeting of diagnostic measures for detecting diabetes and its complications allows a more economical use of the resources of the health system.

Surprisingly, the identification of patients with increased risk of suffering from type 2 diabetes and the determination of the individual's risk of suffering from diabetes could be achieved with a cardiac troponin. Cardiac troponins have been described as useful for the identification and prediction of various cardiovascular diseases. However, they have not been described as causally linked to the development of diabetes. Based on this knowledge it appears likely that cardiac troponins do not represent biomarkers for the type 2 diabetes itself, i.e. increased insulin resistance. Cardiac troponins probably indicate early complications of a yet undefined metabolic condition that finally leads to diabetes.

Consequently, the present invention also relates to a method for the early detection of an early complication of a metabolic condition that leads to future type 2 diabetes comprising the determination of the amount of a cardiac troponin or a variant thereof in a sample of an individual and the comparison of the determined amount of the cardiac troponin or the variant thereof with a reference amount.

The method of the present invention, preferably, comprises the steps of a) determining the amount of a cardiac troponin or a variant thereof in a sample of an individual; b) comparing the determined amount of the cardiac troponin or the variant thereof with a reference amount; and c) diagnosing the early complication of the metabolic condition that leads to future type 2 diabetes.

Accordingly, the present invention relates to a method for the detection of the initial stages of a complication of a metabolic condition that leads to future type 2 diabetes comprising the steps of
a) determining the amount of a cardiac troponin or a variant thereof in a sample of an individual; and
b) comparing the determined amount of the cardiac troponin or the variant thereof with a reference amount to diagnose the early complication of the metabolic condition that leads to future type 2 diabetes.

Also preferably, the present invention relates to a method for the early detection of an early complication of a metabolic condition that leads to future type 2 diabetes comprising the steps of
a) determining the amount of a cardiac troponin or a variant thereof in a sample of an individual;
b) comparing the determined amount of the cardiac troponin or the variant thereof with a reference amount; and
c) diagnosing the early complication of the metabolic condition that leads to future type 2 diabetes based on the comparison of step b).

The "early complication of a metabolic condition that leads to future type 2 diabetes" is, preferably a cardiac disease or disorder. Said disease or disorder is, preferably, characterized by cardiac necrosis.

The "metabolic condition that leads to future type 2 diabetes" is, preferably not characterized by conventional signs and symptoms of increased insulin resistance. Consequently, the individual does, preferably, not suffer from hyperinsulinemia, impaired glucose tolerance as revealed by an oral glucose tolerance test or impaired fasting glycemia.

Advantageously, troponin T, in contrast to NT-pro BNP or other natriuretic peptides has an important role in the identification of cardiac disease in patients at risk of diabetes mellitus or with diabetes mellitus. This information has important diagnostic and therapeutic consequences. Diagnostic consequences include further diagnostic tests such as laboratory tests to assess additional risk factor for cardiovascular, electrocardiography, echocardiography, stress testing and more invasive techniques if needed. Therapeutic measures include improvement of life style, anti-inflammatory drugs tight control of blood pressure and glucose in diabetes mellitus (if present) and cardiovascular invention if needed.

Moreover, the present invention relates to a method for diagnosing a myocardial infarction in a patient based on the determination of the amount of a cardiac troponin in a sample of the patient and the comparison of the determined amount with a reference amount.

It is also provided for a method for diagnosing a myocardial infarction in a patient comprising the steps of
a) determining the amount of a cardiac troponin or a variant thereof in a sample of the patient;
b) comparing the measured amount of the cardiac troponin or the variant thereof to a reference amount;
whereby the results obtained in step b) indicate whether the patient suffers from a myocardial infarction.

Preferably, the method of the present invention comprises the steps of a) determining the amount of a cardiac troponin or a variant thereof in a sample of the patient; b) comparing the measured amount of the cardiac troponin or the variant thereof to a reference amount; and c) diagnosing whether the patient suffers from a myocardial infarction.

A further embodiment of the present invention relates to a method for diagnosing heart failure in a patient comprising the steps of
a) determining the amount of a natriuretic peptide or a variant thereof in a sample of the patient;
b) comparing the measured amount of the natriuretic peptide or the variant thereof to a reference amount;
whereby the results obtained in step b) indicate whether the patient suffers from heart failure.

Preferably, the method of the present invention comprises the steps of a) determining the amount of a natriuretic peptide or a variant thereof in a sample of the patient; b) comparing the measured amount of the natriuretic peptide or the variant thereof to a reference amount; and c) diagnosing whether the patient suffers from a heart failure.

The following examples are merely intended to illustrate the invention. They shall not limit the scope of the claim in any way.

### Example 1: Analytical methods

Troponin T was measured with an immunoassay to be used with the Elecsys and COBAS analyzers from Roche Diagnostics, Mannheim, Germany. The test is capable of measuring troponin T-concentrations from 2 pg/ml to 10000 pg/ml. The assay is based on the sandwich principle and comprises two monoclonal troponin-T specific antibodies. The first of these is biotinylated and the second one is labeled with a Tris(2,2'-bipyridyl)ruthenium(TT)-complex. In a first incubation step both antibodies are incubated with the human serum sample. A sandwich complex comprising cardiac troponin T and the two different antibodies is formed. In a next incubation step streptavidin-coated beads are added to this complex. The beads bind to the sandwich complexes. The reaction mixture is then aspirated into a measuring cell where the beads are magnetically captured on the surface of an electrode. The application of a voltage then induces a chemiluminescent emission from the ruthenium complex which is measured by a photomultiplier. The amount of light is dependent on the amount of sandwich complexes on the electrode.

NT-proBNP was tested with a sandwich immuno-assay using a COBAS analyser from HTACHI-ROCHE. The principle of the test was the same as described above for the troponin-T-immunoassay. The test is capable of measuring NT-proBNP concentrations from 5 pg/ml to 35000 pg/ml.

The GDF-15 assay contains antibodies obtained from R&D systems, Minneapolis, USA and the test is adopted to the assay described by Wollert et al in Clinical Chemistry 53, 2, 284 - 291, 2007. The measuring range of the test is between 300 and 20000 pg/ml.

H-FABP was determined by using the HBT ELISA Test Kit for human heart type fatty acid binding protein (HyCult Biotechnology, Uden, The Netherlands). The test is a solid-phase enzyme-linked immunosorbent assay based on the sandwich principle. The microwells have been pre-coated with monoclonal antibody recognizing human H-FABP. Diluted human serum samples or standards are incubated together with a peroxidase conjugated second antibody (tracer) in the microwells for the incubation period, during which human H-FABP is bound to the solid phase. the tracer antibody is bound to the solid phase. Color develops proportionally to the amount of H-FABP in the sample. The enzyme reation is stopped by the addition of citric acid, the absorbance at 450 nm is measured spectrophotometrically. A standard curve is obtained, from which unknown H-FABP concentrations can be determined.

### Example 2: NT-proBNP, GDF-15, Troponin T and H-FABP in different groups of patients

A group of 149 clinically healthy individuals (51 males, mean age 40 years, range 20-52 years; 99 females, mean age 41 years, range 18 to 56 years) served as an apparently healthy control group. All subjects had normal blood pressure, a normal electrocardiogram, no diabetes mellitus or history of cardiac disease of any type and no cardiovascular risk factors such as smoking. In addition they had normal kidney function, i.e. a glomerular filtration rate higher than 60 ml per minute and 1.73 m² body surface or by a serum creatinine value lower than 1.3 mg/dl.

A further group consisted of 234 clinically stable patients with coronary artery disease (122 males, 112 females, mean age 51.2 years). Coronary artery disease was confirmed by angiography. 82 patients had 1-vessel disease, 60 patients had 2-vessel disease and 92 patients had 3-vessel disease as defined by a reduction of the vessel diameter by at least 50 %. The kidney function of the patients was normal.

Furthermore, 52 patients (38 males, 14 females, mean age 54 years) with stable type 2 diabetes and without evidence of coronary heart disease or signs and symptoms of heart disease were studied. All patients had microalbuminuria (20-300 mg/24 h) but normal kidney function.

| **Table 1A: Biomarkers in apparently healthy subjects** | | | | | | |
|---|---|---|---|---|---|---|
| Percentile | NT-pro-BNP [pg/ml] | GDF-15 [pg/ml] | Troponin T [pg/ml] | Troponin T/ NT-proBNP | H-FABP [pg/ml] | H-FABP/ NT-proBNP |
| 25^{th} | 18.45 | 503 | 0.0* | 0 | 1257 | 68 |
| 50^{th} | 37.25 | 580 | 0.0* | 0 | 1483 | 40 |
| 75^{th} | 67.58 | 683 | 0.0* | 0 | 1793 | 27 |
| 95^{th} | 151.75 | 925 | 2.9 | 0.02 | 2418 | 16 |

| | | | | | | |
|---|---|---|---|---|---|---|
| *Troponin T amounts below 2.0 pg/ml could not be determined reliably. They are, thus indicated as zero. | | | | | | |

| **Table 1B: Biomarkers in patients with coronary artery disease not suffering from diabetes** | | | | | | |
|---|---|---|---|---|---|---|
| Percentile | NT-pro-BNP [pg/ml] | GDF-15 [pg/ml] | Troponin T [pg/ml] | Troponin T/ NT-proBNP | H-FABP [pg/ml] | H-FABP/ NT-proBNP |
| 25^{th} | 95.5 | 515 | 1.2 | 0.01 | 1548 | 16 |
| 50^{th} | 266.0 | 690 | 6.6 | 0.02 | 2236 | 8 |
| 75^{th} | 928.0 | 1045 | 23.0 | 0.02 | 3380 | 4 |

| **Table 1C: Biomarkers in patients with type 2 diabetes and a cardiac disorder** | | | | | | |
|---|---|---|---|---|---|---|
| Percentile | NT-pro-BNP [pg/ml] | GDF-15 [pg/ml] | Troponin T [pg/ml] | Troponin T/ NT-proBNP | H-FABP [pg/ml] | H-FABP/ NT-proBNP |
| 25^{th} | 14.5 | 1021 | 5.8 | 0.40 | 2723 | 188 |
| 50^{th} | 35.0 | 1583 | 10.7 | 0.31 | 3447 | 98 |
| 75^{th} | 77.8 | 2080 | 17.0 | 0.22 | 4330 | 56 |

As shown by the increased amounts of troponin T and the normal amounts of NT-proBNP, NT-proBNP is apparently not useful for ruling out a cardiac disease in a diabetes patient. Exclusion of heart disease in this group of patient as NT-pro BNP values were within the normal range whereas GDF 15 levels were indicative of heart failure. Troponin T levels were however significantly elevated when compared with a group of healthy subjects and reached even higher levels when compared to a group of patients with established and documented coronary artery disease. Thus Troponin T which is an established indicator of cardiac necrosis confirmed cardiac disease in the diabetes mellitus patient group. This was confirmed by echocardiography in a subgroup of patients, these patients showed echocardiographic signs of diastolic dysfunction. Data obtained for H-FABP which is a marker of impaired cardiac metabolism were similar to those of troponin T. Thus while NT-proBNP and GDF 15 fail to establish a clear diagnosis of cardiac disease in diabetes mellitus patients because they provide contradictory results, these discrepancies are resolved by troponin T and H FABP which in case of elevation provide a reliable indication of existing heart disease which preferably in characterized as diastolic dysfunction by echocardiography.

The high troponin T amounts strongly indicate ongoing cardiac necrosis. GDF-15 is much higher in diabets patients when compared to healthy individuals or the patients with coronary artery disease but without diabetes. Thus, cardiac troponin T and H-FABP remain as the only markers that are useful for diagnosing cardiac diseases in diabetes patients.

### Example 3: Troponin T and H-FABP during therapy with anti-inflammatory drugs

Ibersatan belongs to the group of angiotensin receptor blockers, these drugs are as ACE inhibitors widely used in diabetes and heart failure patients to suppress the actviation of the renin angiotensin system which is activated in both disorders in order to slow down disease progress specifically in the heart and the kidney. Treatment effects can be seen if markers of cardiac function (NT-pro BNP), underlying cardiac disease (GDF 15) or cardiac necrosis or function (troponin T or H FABP) change. In this case troponin T appears to reflect best benefit of ARBs.
The group of patients with type 2 diabetes received 900 mg Irbesartan per day for two months. After this time a decrease of the amounts of troponin T and H-FABP could be observed (table 2). Therapy with an anti-inflammatory drug, thus, improves the condition of these patients. It can be expected that a longer course of treatment would cause a stronger decrease. The levels of the markers at baseline are given in table 1C.

| **Table 2: Biomarkers in patients with type 2 diabetes after treatment with Irbesartan** | | | | |
|---|---|---|---|---|
| Percentile | NT-pro-BNP [pg/ml] | GDF-15 [pg/ml] | Troponin T [pg/ml] | H-FABP [pg/ml] |
| 25^{th} | 10.5 | 1121 | 4.9 | 2535 |
| 50^{th} | 33.4 | 1414 | 8.9 | 3117 |
| 75^{th} | 74.0 | 1818 | 15.7 | 4110 |

### Example 4: Troponin T and GDF-15 for the prediction of type 2 diabetes

Between 1982 and 1984 a population of apparently healthy individuals was randomly recruited. At entry these individuals were 30, 40, 50 or 60 years old. In 1994 a subgroup of these individuals was re-examined and blood samples were taken at this point. Individuals with established cardiovascular disease (established heart failure or established cardiovascular disease) or diabetes mellitus were excluded from further analysis. Median NT-proBNP levels were 46 pg/ml (21-83 pg/ml) for this group of individuals indicating normal cardiovascular function according to the upper limit of normal of the test (125 pg/ml).

Individuals were followed over 12 years with respect to the development of type 2 diabetes as assessed by standard criteria. Standard criteria were:
- fasting glucose above 126 mg/dl or 7 mmol/l;
- random glucose exceeding 200 mg/dl or 11.1 mmol/l; or
- glucose in an oral glucose tolerance test above 200 mg/dl or 11.1 mmol/l.

| **Table 3: Troponin T for the prediction of type 2 diabetes** | |
|---|---|
| Troponin T | Patients with type 2 diabetes after 12 years [%] |
| Below 3 pg/ml | 5.9 |
| Above 3 pg/ml | 13.1 |

| **Table 4: GDF-15 for the prediction of type 2 diabetes** | | |
|---|---|---|
| Quartile | GDF-15 [pg/ml] | Patients with type 2 diabetes after 12 years [%] |
| 1^{st} | < 426 | 3.9 |
| 2^{nd} | 427-564 | 7.1 |
| 3^{d} | 565-774 | 11.2 |
| 4^{th} | > 775 | 13.6 |

Amongst 2176 individuals tested 186 individuals developed type 2 diabetes during the 12 years.

As can be seen in table 3, those individuals with more than 3 pg/ml troponin T at baseline had more than double the risk of developing type 2 diabetes as compared to those with troponin T amounts below 3 pg/ml.

Similarly, GDF-15 levels at baseline predicted the risk of developing type 2 diabetes during the course of the study (table 4). Individuals in the fourth quartile had a more than threefold increased risk of developing diabetes as compared to individuals in the first quartile.

The finding that troponin T allows a risk prediction with respect to the development of type 2 diabetes was surprising because troponins are cardiac specific molecules that are related to cardiac cell death but not involved in the pathogenesis of type 2 diabetes.GDF-15 supported the risk stratification as is shown in table 5 for the combination of GDF-15 and troponin T.

| Table 5: Percentage of patients with type 2 diabetes after 12 years based oon GDF-15 and troponin T | | |
|---|---|---|
| | GDF-15 below median | GDF-15 above median |
| Troponin T < 3 pg/ml | 4.2 % | 7.1 % |
| Troponin T > 3pg/ml | 11.1 % | 15.3 % |

Patients with troponin T above 3 pg/ml and GDF-15 above the median had an almost fourfold higher risk of developing type 2 diabetes as compared to the patients in the group with the lowest risk.

### Example 5: GDF-15 and troponin T as predictors of the development of diabetes in patients suffering from the metabolic syndrome

Amounts of troponin T and GDF-15 were determined in 420 patients (261 females, 159 males, mean age 48 years) suffering from the metabolic syndrome. The patients were followed for 12 years with respect to the development of type 2 diabetes. 97 out of 400 patients developed type 2 diabetes. Data for 20 patients are missing.

For diagnosis of the metabolic syndrome the EGIR criteria (European Group for the Study of Insulin Resistance) were applied. Patients were included into the study if they had serum insulin levels larger than 44 pmol/l and additionally fulfilled at least two of the following criteria:
- BMI above 30 kg/m2
- triglyceride levels above 2 mmol/l or HDL cholesterol below 1 mmol/l
- fasting glucose above 6.1 mmol/l
- systolic blood pressure above 140 mmHg and diastolic blood pressure above 90 mmHg

| **Table 6: GDF-15 for the prediction of type 2 diabetes in patients with metabolic syndrome** | | |
|---|---|---|
| Quartile | GDF-15 [pg/ml] | Patients with type 2 diabetes after 12 years [%] |
| 1^{st} | <488 | 15 |
| 2^{nd} | 488-656 | 28 |
| 3^{d} | 656-863 | 27 |
| 4^{th} | > 863 | 29 |

| **Table 7: Troponin T for the prediction of type 2 diabetes in patients with metabolic syndrome** | | |
|---|---|---|
| Quartile | Troponin T [pg/ml] | Patients with type 2 diabetes after 12 years [%] |
| 1^{st} | < 1.0 | 22 |
| 2^{nd} | 1.0-3.7 | 23 |
| 3^{d} | 3.7-7.3 | 26 |
| 4^{th} | > 7.3 | 27 |

Table 6 shows that the 25th percentile of GDF-15 can be used to identify patients with an increased risk of developing type 2 diabetes over the observational period. In contrast to the findings in healthy individuals (see example 4) troponin T is less useful for the prediction of future type 2 diabetes in patients suffering from the metabolic syndrome.

Data obtained in selected patients with the metabolic syndrome (MS) indicate that the risk to develop type 2 diabetes nearly triples ( 24.3 % versus 8.5 %) when compared to the general population. In addition to this, median GDF 15 und median Troponin T levels were higher in the group with MS than in the general population. While GDF 15 retained its predictive power for the development of diabetes mellitus both in the general population as well as in the MS group, Troponin T was a stronger predictor for futures diabetes mellitus in the general population than in the MS group although differences in risk reaches statistical significance (p under 0.05). As MS is composed of a variety of different risk factors, factors other than those preceding diabetes are apparently responsible for Troponin T elevations in the metabolic syndrome, thus weakening the predictive power of Troponin T for type 2 diabetes mellitus in the metabolic syndrome group.

### Example 6: General conclusions

Thanks to the present invention cardiac disorder can be diagnosed in patients with type 2 diabetes mellitus in whom established markers such as NT-pro BNP (false negative) and GDF 15 (false positive) fail to diagnose a cardiac disorder. Troponin T as a marker of cardiac necrosis and Heart fatty acid binding protein as a marker of impaired cardiac metabolism however facilitate such a diagnosis. Moreover, Troponin T was found to be an indicator of future type 2 diabetes mellitus in the general population and to a far lesser extent in metabolic syndrome (together with GDF 15). Surprisingly, cardiac necrosis was apparent in patients before type 2 diabetes mellitus developed suggesting that processes causing cardiac damage are ongoing even before type 2 diabetes mellitus develops. The detection of cardiac necrosis and thus cardiac disease which is clinically not apparent is important and triggers the need for more intense monitoring of the patients, the introduction of life style changes and, even more importantly, early drug intervention. Drug intervention includes primarily the application of statins, ACE inhibitors, Angiotensin receptor blockers and potentially aldosterone antagonists. In addition, it requires consideration of additional diagnostic procedures such as ECG, echocardiography, CT or MRI scan and ultimately invasive methods such as angiography, in order to identify pathological changes requiring treatment.

## Claims

1. A method for diagnosing a cardiac disorder in a diabetes patient with amounts of a natriuretic peptide or variant thereof and GDF-15 or a variant thereof that provide contradictory information comprising the steps of
a) determining the amount of a cardiac troponin or a variant thereof and/or H-FABP or a variant thereof in a sample of the patient; and
b) comparing the measured amounts of the markers to reference amounts to diagnose the cardiac disorder.

2. The method of claim 1, wherein the cardiac troponin is troponin T.

3. The method of claim 1 or 2, wherein the natriuretic peptide is NT-proBNP.

4. The method of any of claims 1 to 3, wherein the cardiac disorder is a diastolic dysfunction.

5. The method of any of claims 2 to 4, wherein the reference amount for the cardiac troponin is 10.7 pg/ml and the reference amount for H-FABP is 3400 pg/ml.

6. The method of any of claims 1 to 4, wherein the ratio of the amount of the cardiac troponin or the variant thereof to the amount of the natriuretic peptide or the variant thereof and the ratio of the amount of H-FABP or the variant thereof to the amount of the natriuretic peptide or the variant thereof is additionally calculated in step a) and said ratios are compared to reference values in step b).

7. A method for monitoring the treatment of a cardiac disorder with an anti-inflammatory drug in a diabetes patient having amounts of a natriuretic peptide or variant thereof and GDF-15 or a variant thereof that provide contradictory information comprising the steps of
a) determining the amount of a cardiac troponin or a variant thereof and/or H-FABP or a variant thereof;
b) repeating step a) in at least one further sample of the patient;
c) comparing the amounts determined in the first and the at least one further sample to assess whether the treatment of the cardiac disorder was successful.

8. A method for predicting in an individual the risk of suffering from diabetes , preferably diabetes type 2, comprising the steps of
a) determining in a sample of the individual the amount of a cardiac troponin or a variant thereof;
b) comparing the determined amount of the cardiac troponin or the variant thereof to determine the risk of the individual to suffer from diabetes.

9. The method of claim 6, wherein the amount of GDF-15 or a variant thereof isadditionally determined in step a) and compared to a reference amount in step b).

10. Use of an antibody binding cardiac troponin or a variant thereof and/or an antibody binding H-FABP or a variant thereof for diagnosing a cardiac disorder in a diabetes patient having amounts of a natriuretic peptide or variant thereof and of GDF-15 or a variant thereof that provide contradictory information.

11. Use of an antibody binding cardiac troponin or a variant thereof for predicting the risk of an individual to suffer from type 2 diabetes.

12. A method for diagnosing a myocardial infarction in a patient comprising the steps of
a) determining the amount of a cardiac troponin or a variant thereof in a sample of the patient; and
b) comparing the determined amount of the cardiac troponin or the variant thereof to a reference amount to diagnose the myocardial infarction.

13. A device for diagnosing a cardiac disorder in a diabetes patient having amounts of a natriuretic peptide or variant thereof and GDF-15 or a variant thereof that provide contradictory information comprising
a) an analyzing unit for determining the amount of a cardiac troponin or a variant thereof and/or the amount of H-FABP or a variant thereof in a sample of the patient; and
b) an evaluation unit comprising means for comparing the measured amount of the cardiac troponin or the variant thereof and/or of H-FABP or the variant thereof to a reference amount.

14. A kit for diagnosing a cardiac disorder in a diabetes patient having amounts of a natriuretic peptide or variant thereof and GDF-15 or a variant thereof that provide contradictory information comprising
a) an analyzing agent for determining the amount of a cardiac troponin or a variant thereof and/or the amount of H-FABP or a variant thereof in a sample of the patient; and
b) an evaluation unit comprising means for comparing the measured amount of the cardiac troponin or the variant thereof and/or of H-FABP or the variant thereof to a reference amount.
